# EUROPEAN PATENT SPECIFICATION

(11) **EP 2 173 336 B1**
(45) Date of publication and mention of the grant of the patent: **30.03.2016**
(21) Application number: 08767263.0
(22) Date of filing: 27.06.2008
(51) Int. Cl.: A61K 31/194, A61K 31/198, A61P 9/14, A61P 9/12, A61P 27/02, A61P 1/00, A61K 38/05, A61K 38/06, A23L 33/10

(54) **Alpha-ketoglutaric acid for use in the treatment of hypertension or pulmonary hypertension**
Alpha-Ketoglutarsäure für die Verwendung in der Behandlung von Hypertonie oder pulmonarer Hypertonie
L'acide alpha-cétoglutarique destinée à être utilisée dans le traitement de l'hypertension artérielle ou de l'hypertension artérielle pulmonaire

(30) Priority: 02.07.2007 SE 0701602
(43) Date of publication of application: 14.04.2010
(73) Proprietor: Protista Biotechnology AB, 267 34 Bjuv (SE)
(72) Inventor: PIERZYNOWSKI, Stefan, G., S-211 14 Malmö (SE)
(74) Representative: Novitas Patent AB
(86) International application number: PCT/SE2008/050797
(87) International publication number: WO 2009/005464

(56) References cited:
- WO-A1-95/34301
- WO-A1-2005/123056
- WO-A1-2006/046880
- DE-A1- 19 929 993
- WIESNER R J ET AL: "Pathways of succinate formation and their contribution to improvement of cardiac function in the hypoxic rat heart" BIOCHEMICAL MEDICINE AND METABOLIC BIOLOGY, ACADEMIC PRESS LNKD- DOI:10.1016/0885-4505(88)90100-4, vol. 40, no. 1, 1 August 1988 (1988-08-01) , pages 19-34, XP022954443 ISSN: 0885-4505 [retrieved on 1988-08-01]
- VATURI MORDEHAY ET AL: "Transthoracic echocardiographic assessment of proximal ascending aorta elasticity in familial heterozygous hypercholesterolemia patients." THE ISRAEL MEDICAL ASSOCIATION JOURNAL : IMAJ JUL 2003 LNKD- PUBMED:12901240, vol. 5, no. 7, July 2003 (2003-07), pages 475-478, XP008127584 ISSN: 1565-1088
- KJELLMAN U.W. ET AL.: 'Addition of alpha-ketoglutaric acid to blood cardioplegia improves cardioprotection' ANN. THORAC. SURG. vol. 63, 1997, pages 1625 - 1634, XP003023863
- "Arterial Hypertension" In: Beers, Mark H.: "The Merck Manual of Diagnosis and Therapy", 2006, Merck Research Laboratories, Whitehouse Station, NJ pages 604-621,

## Description

### Field of the invention

The present invention relates to a new use of known pharmacologically active chemical compounds. More particularly, the present invention relates to the new use of alpha-ketoglutarate, and pharmaceutically acceptable salts thereof for use in the treatment and/or prophylaxis of hypertension or pulmonary hypertension.

### Background

### Gastric surgery

With a steady increase in obesity in the Western world, the prospect of invasive forms of weight reduction as a mean of reducing mortality and improving co-morbid conditions has become increasingly attractive. Indeed, some would advocate it as being the only acceptable form of weight loss since an NIH statement released in 1992 reported that non-operative approaches have a success rate of 5% or less in obese patients. Gastric bypass procedures have been shown to result in up to 70% loss of excess body weight, with long-term weight loss lasting from 10-14 years.

Recently, Coates and her colleagues reported that such invasive procedures as the gastric bypass, appears to have an impact on skeletal health. In a study of 25 patients receiving gastric bypass surgery, markers of bone turnover were found to be significantly elevated, and bone mineral density was decreased in the hip trochanter.

Gastric bypass surgery is also known to be beneficial in terms of reducing hypertension, a problem commonly found in the morbidly obese. In a follow-up of 67 morbidly obese patients who had been registered as hypertensive (bp > 160/90 mm Hg), gastric bypass surgery was shown to resolve the preoperative hypertension in 44 individuals (66%).

### Blood vessel elasticity in disease

It has been known for quite some time that blood vessel elasticity, in particular arterial elasticity is involved in the disease condition hypertension and related conditions. It has been shown that aortic stiffness is an independent predictor of progression to hypertension in nonhypertensive subjects, i.e. that aortic stiffness is a risk factor for developing hypertension that is independent of other known risk factors (Dernellis and Panaretou, Hypertension. 2005;45:426-431.). Retinal vascular disease may be due to hypertensive retinopathy, in turn caused by systemic hypertension.

Pulmonary hypertension (PH) is an increase in blood pressure in the pulmonary artery, pulmonary vein, or pulmonary capillaries, together known as the lung vasculature, leading to shortness of breath, dizziness, fainting, and other symptoms, all of which are exacerbated by exertion. Depending on the cause, pulmonary hypertension can be a severe disease with a markedly decreased exercise tolerance and may lead to right-sided heart failure. Pulmonary arterial hypertension (WHO Group I) involves the vasoconstriction or tightening of blood vessels connected to and within the lungs. This makes it harder for the heart to pump blood through the lungs. Over time, the affected blood vessels become both stiffer and thicker, in a process known as fibrosis. This further increases the blood pressure within the lungs and impairs their blood flow. In addition, the increased workload of the heart causes may cause right ventricular hypertrophy which may progress to right ventricular failure.

Aneurysm (or aneurism) is a localized, blood-filled dilation (balloon-like bulge) of a blood vessel caused by disease or weakening of the vessel wall. Aneurysms most commonly occur in arteries at the base of the brain (the circle of Willis) and in the aorta (the main artery coming out of the heart), a so-called aortic aneurysm. The bulge in a blood vessel can burst and lead to illness or death at any time. The larger an aneurysm becomes, the more likely it is to burst.

Although treatments (medical and surgical) for hypertension, pulmonary hypertension, ventricular hypertrophy and aneurysms exist, there is still a need for improved treatments having better efficacy and/or fewer side effects and/or better cost-effectiveness.

### Summary of the invention

The inventor has now shown that the elastic properties of the arteries of obese individuals change after they have undergone gastric surgery. A bypass operation changes the uptake of nutrients affecting the structure of arteries, in a similar fashion to that seen in the skeletal system, with adverse consequences for their elasticity and strength. However, no effective treatment of these negative effects has been reported. Hence, an unmet need to prevent these negative effects of stomach operation was identified. Furthermore, it was also found that the same treatment may be used in other subjects in need of improved blood vessel elasticity, for example elderly subjects. The present invention relates to the following items:
1. A substance selected from the group consisting of:
   a. alpha-ketoglutaric acid (AKG) and
   b. pharmaceutically acceptable salts of alpha-ketoglutaric acid;
   for use in the treatment and/or prophylaxis of hypertension or pulmonary hypertension.
2. A substance for use according to item 1, wherein the substance is for use in a subject having undergone gastric surgery.
3. A substance for use according to item 2, wherein the gastric surgery is gastric bypass surgery, gastrectomy, partial gastrectomy or gastric banding.
4. A substance for use according to any of the above items, wherein the substance is for use in a subject suffering from conditions involving malnutrition.
5. A substance for use to any of the above items, wherein the substance is for use in a subject being elderly.
6. A substance for use according to any of the above items, wherein the substance is alpha-ketoglutaric acid or an alkali or alkaline earth metal salt thereof.
7. A substance for use according to item 6, wherein the substance is sodium alpha-ketoglutarate.
8. A substance for use according to item 6, wherein the substance is calcium alpha-ketoglutarate.
9. A substance for use according to any of the above items, wherein the dosage to be given to a patient is in the interval from 1 to 1000 mg/kg body weight/day.
10. A substance for use according to any of the above items, wherein the dosage to be given to a patient is in the interval from 10 to 400 mg/kg body weight/day.
11.A substance for use according to any of the above items, wherein the dosage to be given to patients is in the interval from 10 to 100 mg/kg body weight/day.

### Brief description of the drawings

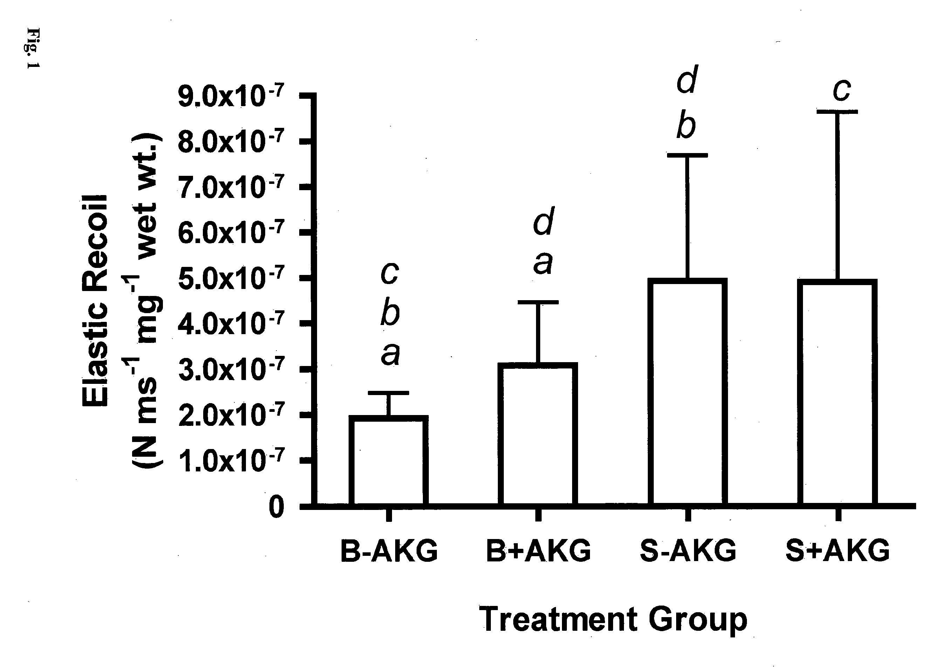

The present invention will be further explained in the following description with the aid of preferred embodiments, example studies and accompanying drawings of which
Figure 1 shows elastic recoil recordings from aorta sections of bypass (B) and sham operated rats (S) administered AKG (+AKG) or vehicle (-AKG). Recordings were made to a force transducer attached to an A/D converter at a sampling rate of 1,000 samples/s. Each point represents the mean ± SE. Significant differences between the means are given: a & d = p<0.05 and b = p<0.01, c = p=0.01. Animals allocated to the four groups were as follows; B-AKG n=6, B+AKG n=11, S-AKG n=12 and S+AKG n=12.

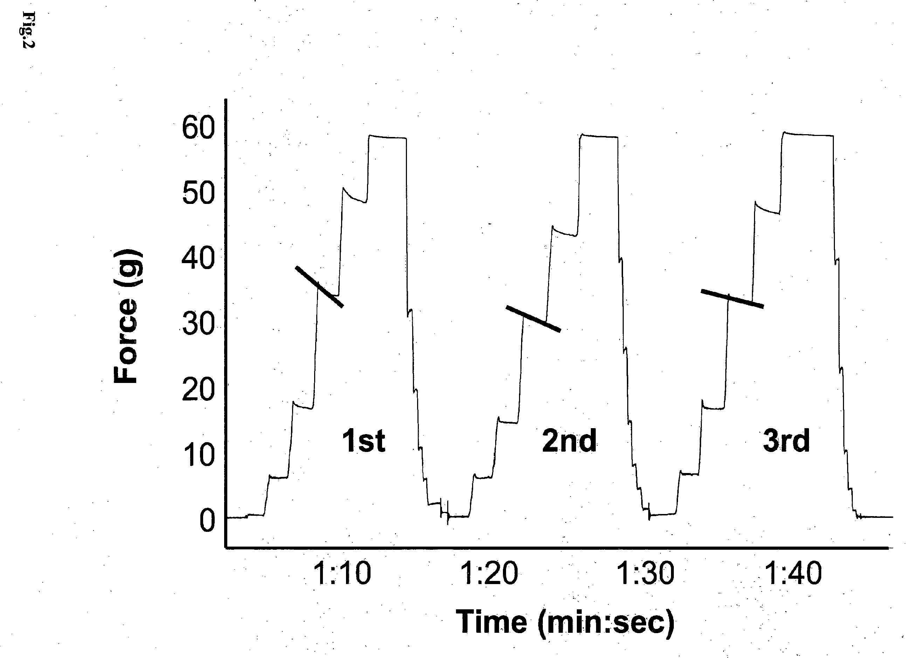

Figure 2 shows a typical experimental trace for an aorta section exposed to a series of stretch and relaxation cycles. The maximum stretch applied was approximately 0.14% of that measured in rat aorta (range 13-14 kPa). Note the slope of the line for the 1 st stretch/relaxation cycle, compared to the slope of the lines for the 2nd and 3rd cycles. This slope represents the elastic recoil (approx. 16% of the manually applied tension) inherent in the aorta section. Clearly repeated stretch/relaxation cycles in this range results in reduced elasticity

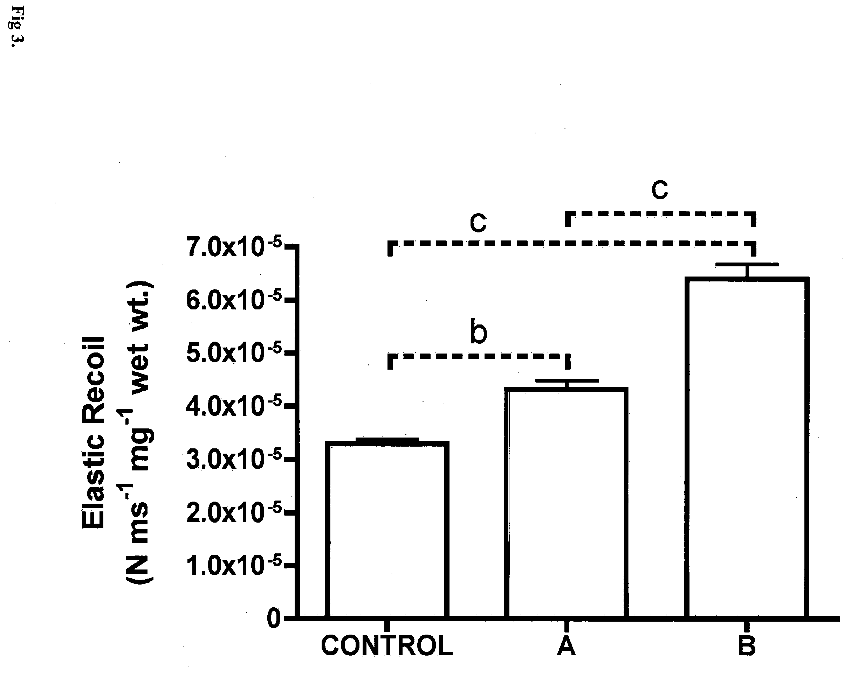

Figure 3 shows a first stretch series: Elastic recoil recordings from aorta sections of CONTROL and (A) Na-AKG as well as (B) Ca-AKG treated mice. Recordings were made to a force transducer attached to an A/D converter at a sampling rate of 1,000 samples/s. Each point represents the mean ± SE. Significant differences between the means are given: a = P<0.05 and b = P<0.01, c = P<0.001. Animals allocated to the three groups were n = 6 for all.

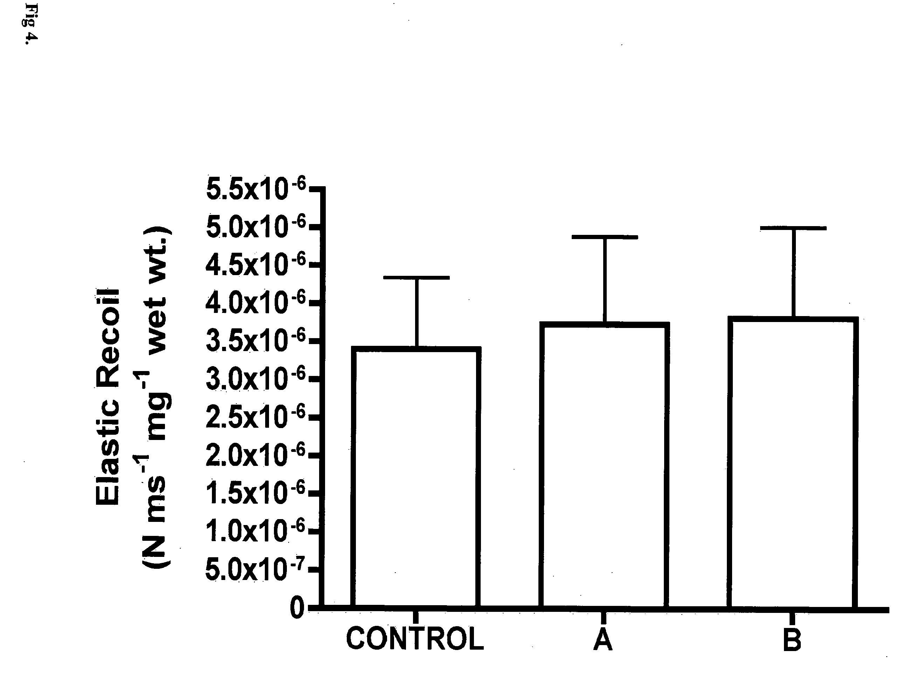

Figure 4 shows a second stretch series: Elastic recoil recordings from aorta sections of CONTROL and (A) Na-AKG as well as (B) Ca-AKG treated mice. Recordings were made to a force transducer attached to an A/D converter at a sampling rate of 1,000 samples/s. Each point represents the mean ± SE. Significant differences between the means are given: a = P<0.05 and b = P<0.01, c = P<0.001. Animals allocated to the three groups were n = 6 for all.

### Detailed description

The present disclosure describes an effective and safe treatment that can be used to improve blood vessel elasticity in a subject in need thereof. In particular, the blood vessels whose elasticity is improved are arteries, but elasticity of veins, capillaries, venules and arterioles may also be improved by the invention.

As there is an established link between blood vessel elasticity and in particular arterial elasticity to hypertension and pulmonary arterial hypertension the described treatment improving blood vessel elasticity can be used in the manufacture of a medicament for the treatment and/or prophylaxis of hypertension and pulmonary arterial hypertension. In turn, hypertension is known to be a causative factor in cardiovascular disease, retinal vascular disease, heart failure, stroke, atherosclerosis, kidney failure, nephrosclerosis and other diseases. Similarly, pulmonary arterial hypertension is known to be a causative factor in right ventricular hypertrophy.

The term "treatment" in its various grammatical forms in relation to the present invention refers to preventing, curing, reversing, attenuating, alleviating, ameliorating, inhibiting, minimising, suppressing, or halting the negative effects of the condition being treated.

The term "negative effects" in relation to gastric surgery in the context of the present invention refers to the adverse impact on blood vessel performance, e.g. arterial elasticity and/or strength, seen following gastric surgery. For example a decreased elasticity of the arteries is seen following a gastric bypass operations.

The term "malnutrition" means a medical condition caused by an improper or insufficient diet, typically resulting from inadequate consumption, poor absorption, or excessive loss of nutrients.

Certain conditions related to malnutrition appear despite a proper diet. For example, gastrointestinal tract function may become impaired because of old age or other sickness. In such cases the impaired digestion may be due to e.g. lack of or improper production of host digestive enzymes in stomach, intestine, pancreas, etc.; inadequate bile production; inadequate gastric pH (impaired HCl production); or other causes. Villar atrophy due to destruction of the villi by aging, diet (e.g. gluten intolerance) or a disease may be a direct cause for malnutrition due to impaired absorption. Conditions involving bacterial overgrowth or lack of gut bacteria can also be a reason for malnutrition. There are several additional causes for malnutrition e.g., gut cancers, surgery, toxins, genetic, circulatory (blood and lymph) problems, anorexia, etc. In any case, malnutrition and conditions related to malnutrition result in kachexia and lowering down of vital functions.

The term "elderly" in the context of the invention means a chronological age where age-related degeneration of the organism (e.g human) is starting to become evident. In case of humans, elderly may be defined as being over 40 years of age, preferably over 50 years of age, more preferably over 60 years of age, or most preferably over 65 years of age.

By "improving blood vessel elasticity" is meant that the elasticity of the blood vessels becomes greater, i.e. the vessels become less stiff. The term also encompasses increased tensile strength of the vessels.

Examples of gastric surgery encompassed in relation to the present invention include but are not limited to gastric bypass surgery, gastrectomy, partial gastrectomy, and gastric banding.

Thus according to one aspect of the present invention, there is provided the new use of at least one member selected from the group consisting of alpha-ketoglutaric acid, and pharmaceutically acceptable salts thereof, for the manufacture of a pharmaceutical preparation or a food or feed supplement for the treatment and/or prophylaxis of hypertension, or pulmonary arterial hypertension.

In one embodiment, the pharmaceutical preparation is directed to subjects that have undergone gastric surgery.

In one embodiment, the pharmaceutical preparation is directed to subjects that suffer from conditions involving malnutrition.

In one embodiment, the pharmaceutical preparation is directed to subjects that are elderly.

According to one embodiment of the invention alpha-ketoglutaric acid or an alkali or alkaline earth metal salt thereof or a combination thereof is used.

Preferably sodium alpha-ketoglutarate is used. Even more preferably, calcium alpha-ketoglurate is used. Sodium alpha-ketoglutarate provides faster uptake after enteral administration with higher peak blood levels, whereas calcium alpha-ketoglutarate provides slowed uptake with longer lasting effect after enteral administration. Example 2 shows that calcium alpha-ketoglutarate gives better effect in certain conditions than sodium alpha-ketoglutarate.

The pharmaceutical preparations of the active principle or principles used in accordance with the present invention may be administered to a vertebrate, including mammals and birds, such as rodent, such as a mouse, rat, guinea pig, or a rabbit; a bird, such as a turkey, hen or chicken and other broilers and free going animals; a cow, a horse, a pig or piglet and other farm animals, a dog, a cat and other pets, and in particular humans.

Administration may be performed in different ways depending on which species of vertebrate to treat, on the condition of the vertebrate in the need of said methods, and on the specific indication to treat.

In one embodiment, the administration is done as a food or feed supplement, such as a dietary supplement and/or a component in form of solid food and/or beverage. Further embodiments may be in suspensions or solutions, such as a beverage further described below. Also, the formats may be in capsules or tablets, such as chewable or soluble, e.g. effervescent tablets, as well as powder and other dry formats known to the skilled man in the art, such as pellets, such as micropellets, and grains.

The administration may be as a parenteral, rectal or oral food or feed supplement, as revealed above. Parenteral vehicles include sodium chloride solution, Ringer's dextrose, dextrose and sodium chloride, lactated Ringer's or fixed oils.

The food and feed supplement may also be emulsified. The active therapeutic ingredient or ingredients may then be mixed with excipients, which are pharmaceutically acceptable and compatible with the active ingredient. Suitable excipients are, for example, water, saline, dextrose, glycerol, ethanol, or the like and combinations thereof. In addition, if desired, the composition can contain minor amounts of auxiliary substances such as wetting or emulsifying agents, pH, buffering agents, which enhance the effectiveness of the active ingredient.

Different formats of the parental food or feed supplement may be supplied, such as solid food, liquids or lyophilized or otherwise dried formulations. It may include diluents of various buffers (e.g., Tris-HCl., acetate, phosphate), pH and ionic strength, additives such as albumin or gelatine to prevent absorption to surfaces, detergents (e.g., Tween 20, Tween 80, Pluronic F68, bile acid salts). solubilizing agents (e.g., glycerol, polyethyleneglycerol), anti-oxidants (e.g., ascorbic acid, sodium metabisulfite), preservatives (e.g.,Thimerosal, benzyl alcohol, parabens), bulking substances or tonicity modifiers (e.g., lactose, mannitol), covalent attachment of polymers such as polyethylene glycol to the composition, complexation with metal ions, or incorporation of the material into or ontoparticulate preparations of polymeric compounds such as polylactic acid, polglycolic acid, hydrogels, etc, or onto liposomes, microemulsions, micelles, unilamellar or multilamellarvesicles, erythrocyte ghosts, or spheroplasts.

In one embodiment, the food or feed supplement is administered in the form of a beverage, or a dry composition thereof, in any of the methods according to the invention.

The beverage comprises an effective amount of the active ingredient or ingredients thereof, together with a nutritionally acceptable water-soluble carrier, such as minerals, vitamins, carbohydrates, fat and proteins. All of these components are supplied in a dried form if the beverage is provided in a dry form. A beverage provided ready for consumption further comprises water. The final beverage solution may also have a controlled tonicity and acidity, e.g. as a buffered solution according to the general suggestions in the paragraph above.

The pH is preferably in the range of about 2-5, and in particularly about 2-4, to prevent bacterial and fungal growth. A sterilised beverage may also be used, with a pH of about 6-8.

The beverage may be supplied alone or in combination with one or more therapeutically effective composition.

According to a further embodiment the pharmaceutical preparations as drug for oral and rectal use may be in the form of tablets, lozenges, capsules, powders, aqueous or oily suspensions, syrups, elixirs, aqueous solutions and the like comprising the active ingredient or ingredients in admixture with a pharmaceutically acceptable carrier and/or additives, such as diluents, preservatives, solubilizers, emulsifiers, adjuvants and/or carriers useful in the methods and use disclosed in the present invention.

Further, as used herein "pharmaceutically acceptable carriers" are well known to those skilled in the art and may include, but are not limited to, 0.01-0.05M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Preservatives and other additives may also be present, such as, for example, antimicrobials, antioxidants, chelating agents, inert gases and the like.

The dosage to be administered will vary depending on the active principle or principles to be used, the condition to be treated, the age, sex, weight etc. of the patient to be treated but will generally be within the range from 1 to 1000 mg/kg body weight/day, or from 10 to 400mg/kg body weight and day, preferably from 10 to 100 mg/kg body weight/day.

The embodiments listed above may be freely combined with one another. Thus, the details and particulars described above and in the claims apply mutatis mutandis to any other embodiments of the invention. While the invention has been described in relation to certain disclosed embodiments, the skilled person may foresee other embodiments, variations, or combinations which are not specifically mentioned but are nonetheless within the scope of the appended claims.

The expression "comprising" as used herein should be understood to include, but not be limited to, the stated items.

The invention will now be further illustrated by means of examples which should not be construed to limit the scope of the invention.

### Examples

### EXAMPLE 1

This study aims to address, 1) the effect on the elasticity of arteries of a bypass operation in rats, linking the oesophagus to the duodenum, 2) the long-term implications of just such an operation on blood pressure, and 3) any beneficial effect of AKG-intake with respect to reversal of any changes in arterial elasticity arising from a bypass-operation.

### Animals and aorta preparation

Adult male Sprague Dawley rats, housed at the animal facilities of the Department of Comparative Physiology, Lund University, were used. The animals were raised under the same conditions with a 12/12 light cycle, and had a body weight of 479 ± 5 g. Rats were fed rodent pellets ad libitum (Altromin no. 1314 Spezialfutterwerke, Lage, Germany) and given free access to drinking water. Rats were grouped accordingly, Bypass operated without AKG (B-AKG) (n=6), Bypass operated with AKG (B+AKG) (n=11), Sham operated without AKG (S-AKG) (n=12), Sham operated with AKG (S+AKG) (n=12).

The rats were killed by exposure to 95% CO₂ and cervical dislocation. Rats were killed in accordance with local and national guidelines.

A dissected portion of the abdominal aorta, prior to the right- and left common iliac arteries, was carefully cleaned to remove adhering tissues. The aorta was cut into approx. 6-9 mm pieces with a diameter at rest of 3-4 mm, and each piece was then securely attached at one end to a force transducer and at the other to a metal pin on a mounting block, as described in part (Harrison et al. Reprod Fertil Dev. 1997;9(7):731-40, Harrison and Flatman Am J Physiol. 1999 Dec;277(6 Pt 2):R1646-53). The weight of the aorta pieces was in the range of 8-25 mg (median 14.32 mg) with an average diameter of 3.75 ± 0.08 mm.

Aorta sections were immersed into oxygenated and thermostatically controlled chambers (37°C), having an internal depth and diameter of 5.5 and 3.2 cm, respectively, and holding 44 ml of phosphate buffered saline (0.15 M PBS, pH 7.4) comprising in mM; NaCl 136.91, KCl 2.68, Na₂HPO₄ 8.08 and NaH₂PO₄ 1.66. Force was measured using a FTO3 force displacement transducer (Grass Instrument, West Warwick, RI) connected to a home-built bridge amplifier which was interfaced with a 8S PowerLab A/D Converter (ADInstruments, Chalgrove, Oxfordshire, UK). The transducer had a functional range 0-0.05 kg, with a reliable force of 2 mg, equivalent to 0.004% of the functional range. The PowerLab 8S A/D converter was connected to an iBook G4 running Chart5 v.5.4 Software (AD Instruments, Australia). The data recording was at a sampling speed of 40.000 data samples per second (40 KHz) and the input impedance of the amplifier was 200 MΩ differential

### Force measurements

Aorta sections were suspended vertically, and in triplicate. The recorded signal was adjusted to zero for un-tensioned aorta sections with the aid of an offset dial mounted on the pre-amplifier unit. Each aorta section was then exposed to approx. 5 step-wise increases in tension (each step being approx. 0.09 N), until a final maximal tension of 0.49 N (measured using the FT03 Grass Force transducer) was achieved. This final level of tension was by no means close to the physiological maximum force recorded in aorta sections. The aorta sections were then allowed to relax totally before being exposed to repeat step-wise increases in tension a further two more times. Aorta sections were subsequently removed and weighed. A recording speed of 1000 data samples per sec was used.

Immediately after a step-wise increase in tension, the recording trace was seen to fall very slightly as the aorta tissue exerted a degree of elastic recoil. This fall in the recording trace was measured over as a unit of time.

### Tension calculations

LaPlace's equation assumes the tension (T) in the wall of a hollow cylinder is directly proportional to the cylinders radius (r) and the pressure (p) across the wall, caused by the flow inside the cylinder, such that T = p x r. If one assumes that the tension in the wall of the aorta sections is equivalent to that recorded by the force transducer as the result of a manual stretch, then the pressure that would have given rise to such an increase in tension can be calculated using LaPlace's equation and the measured radius of each aorta section.

### Statistical analysis

Data are presented as mean ± SE. Differences between means were tested for statistical significance with the use of a student's paired t-test with an additional test for Gaussian Normal Distribution. Data were found to be normally distributed and to have equal variance. Differences showing a P value >0.05 were considered non-significant.

### Results

### Tension & pressure measurements

Typically, aorta sections were exposed to approx. 0.034 N (0.49 N max. tension / 14.32 mg median tissue wt.) of tension per mg wet weight when fully tensioned. Thus using LaPlace's equation and an average aorta radius of 1.87 mm, the pressure generated was in the order of 0.018 kPa. Such a pressure increase represents 0.18 % of that typically found in human arteries (10 kPa), and approximately 0.14 % of that measured in rats (13-14 kPa) (Carroll et al. 2006; Duka et al. 2006).

An average manual step increase in tension generated 0.09 N (4.95 x 10-3 N/mg wet wt.), and aorta sections were typically found to recoil by 0.015 N, a value that represents approximately 16% of the manually applied tension.

### Sham-operated control rats

The elasticity of the aorta from the controls was significantly higher than in the bypass-operated rats (P=0.007; 1.9 x 10-7 ± 0.2 x 10-7 N ms-1 mg-1 wet wt. versus 4.9 x 10-7 ± 0.8 x 10-7 N ms-1 mg-1 wet wt. for B-AKG and S-AKG groups, respectively). AKG-intake had no effect on this phenomenon in the control group (P=0.44).

### Bypass-operated rats

Both without and with AKG-intake, bypass-operated rats showed a lower elasticity of aorta than the control rats (P=0.037; 3.1 x 10-7 ± 0.4 x 10-7 N ms-1 mg-1 wet wt. versus 4.9 x 10-7 ± 0.8 x 10-7 N ms-1 mg-1 wet wt. for B+AKG and S-AKG groups, respectively). AKG-intake had a significant effect in the bypass-operated rats, increasing the arterial elasticity to a level between the control rats and the bypass-operated (B-AKG); P=0.047; 1.9 x 10-7 ± 0.2 x 10-7 N ms-1 mg-1 wet wt. versus 3.1 x 10-7 ± 0.4 x 10-7 N ms-1 mg-1 wet wt. for B-AKG and B+AKG groups, respectively (see Fig 1).

### Stretch series

In all the arteries studied, the initial stretch series (e.g. application of tension followed by relaxation) led to a decrease in elasticity under subsequent applications of tension. This effect may be compared to the sort of damage that would be expected to arise with a sudden increase in blood pressure (see Fig 2).

### Discussion

The results of this study clearly show that bypass surgery has an adverse impact on arterial elasticity. Moreover, to our knowledge, this is the first time that such a surgical procedure has been documented as resulting in a dramatic change in arterial elasticity. A similar change is also shown to take place with a stretch comparable to a sudden rise in blood pressure.

### Bypass surgery

The bypass-operated rats in this experiment were not obese or in any other way different from the sham-operated control group. Both groups were exposed to surgery, so any stress related to the surgical procedures was common to them both. The groups remained different, however, in terms of the surgical bypass procedure, linking the oesophagus to the duodenum. This type of bypass operation may be compared to the stomach bypass operation of the Roux-en-Y, where most of the stomach and the duodenum are bypassed in a way which allows some gastric (from the proximal part), pancreatic, biliary and duodenal secretion. This is the most common procedure at present when choosing bariatric surgery as the treatment for morbid obesity (Adrian et al 2003).

Stomach bypass surgery is supposed to limit the food intake and thereby prevent obesity. This effect is quite clearly seen (Coates et al 2004, Cowan and Buffington 1998, Fernstrom et al 2006), but the physiological consequences must also be considered.

The stomach is, apart from acting as a reservoir for and performing the mechanical breakdown of food, also a place for digestion and secretion. When the food gets into the stomach, there is a phase where the digestive enzymes from saliva are still working. Lack of this phase in digestion may especially affect the breakdown of starch. Although this would give less energy, it ought not be vital or create major problems in digestion. The same may be said for the mechanical breakdown of food particles in general.

A more severe aspect is the missing secretion of the stomach. The important components here are the enzymes pepsin and lipase, intrinsic factor and hydrochloric acid. Pepsin is vital for protein breakdown, and the enzyme needs HCl to be activated from the secreted pepsinogen to pepsin. Lack of a stomach may therefore have severe consequences for the amino acid intake and cause amino acid deficiency. Another aspect of this is the release of minerals and vitamins bound to enzymes. If the micronutrients are not released, they cannot be absorbed further along the digestive tract. Lipase from the stomach will break down triglycerides, but even if this does not happen, the triglycerides will still be met with lipases from the pancreas, and the need for fatty acids ought to be met.

The regulation of pancreatic secretion or bile will though be less regulated without the stomach, since the acidity of the entered solution in the duodenum regulates this secretion. The stomach also regulates the amount of food let through into the duodenum at any given time. The transport is dependent on content of carbohydrates, proteins or fats, where fats give the smallest amount and carbohydrates the largest amount through at any given time. This mechanism secures an efficient digestion and regulates the speed of movement through the intestine. With a bypass surgery, this mechanism has been abolished, and the general digestion is compromised. With the changed acidity and the change in flow speed, mal-absorption or diminished absorption may occur for certain components.

Vitamin B 12 is released from proteins and intrinsic factor is secreted in the stomach. Intrinsic factor is vital for the absorption of B12 in ileum. Vitamin B12 is normally present in abundance, but suddenly after the operation, it may not be the case. It is also reported that in humans, Vitamin B 12 deficiency is seen in 70% of the bypass-operated patients when they are followed after the operation Lynch et al.2006, Shah et al 2006). The same authors report anaemia which may also partly be due to iron deficiency caused by less release from especially proteins when the environment is less acidic, and partly be due to the vitamin deficiency. Ca and folate deficiency is also reported after bypass surgery (Lynch 2006, Parkes 2006, Shah 2006). If this is due to poor absorption without the duodenum, or if this is due to less availability of the two is not clear. In the present rat study, the duodenum ought to functioning, but the change in flow and pH may affect the breakdown and nutrients all through the rest of the intestine and show a different pattern of absorption. The flow may very well be affected by influx of hyper-osmolar material into the intestine. This is supported by the assumption, that a flow of hyper-osmolar food into the small intestine may be the cause of the 'dumping syndrome' in some patients with stomach bypass (Lynch et al 2006) which may respond with vomiting.

In our study, the stress effect of the operation is taken into account by using sham-operated rats as our control group. Furthermore, none of the rats were obese or hypertensive before the operation, and we may assume that the arteries had normal elasticity prior to the bypass operation.

In humans, a fall in blood pressure following the bypass operation is seen (Coates, Buffington 1998, Fernstrom et al 2006, Foley et al 1992,). This is probably mainly due to the weight loss, but it may also partly be due to a change in metabolism and hormonal balance. The change in arterial elasticity seen in our bypass-operated rats is therefore not very likely caused by hypertension, since a slight drop in blood pressure or staying normo-tensive is to be expected.

Following the operation, our measurements show clearly a decreased elasticity of the arteries.

The questions are now, what happens after the operation? And what may affect the walls of the arteries? Gokce et al (2005) report a long-term improvement of endothelium-dependent flow-mediated vasodilation with weight loss, and this improvement is significantly better in stomach-bypassed patients when compared with weight loss from medical therapy. In our rats, who were not overweight before the operation, we are only looking at effects of changes in the body's access to nutrients and possible changes in metabolic and hormonal balance.

Cations like calcium and potassium, which might affect the blood pressure, are to a high degree bound to the charged proteins, and are most likely present in less than normal concentrations.

The explanation is possibly to be found somewhere else. If the proteins are not broken down or only partly broken down, there will not be enough amino acids to be transported over the intestine. This must affect the protein turnover. In the artery wall the elasticity is partly due to the connective tissue in the walls. This tissue is remodelled all the time, and if damaged, it will be repaired. If there is an insufficient amount of amino acids available for remodelling or repair, the wall will loose its elasticity over time. Another factor adding to this might be a different hormonal balance due to the difference in signals sent from the alimentary tract. This may be due to the same causes as the ones giving an increase in bone turn-over with an increased bone resorption and following decrease in bone mass found in stomach-bypassed humans. Part of the reason could be, that the proteins necessary for healthy turn-over cannot be digested by the stomach-bypassed rat. The higher turn-over of bone speaks also for a hormonally regulated cycle which has been reprogrammed. If the elasticity is diminished, it is either the structure or the percentage of elastic fibres which gives the overall change.

### Stretch effect

The fluid pressure P within a vessel wall is balanced by the tension T of the wall, divided by the radius of curvature r and the external pressure pn, such that P = pn + T(1/r), as defined according to Laplace's law. If one chooses to neglect external pressure and any support from surrounding tissues, dealing only with a cylindrical vein or artery of a reasonable size, then the equation can be reduced to P = T / r. Furthermore it is well known that the tension developed depends upon the thickness of the vessel wall, that is to say the amount of membrane and muscular tissue comprising the wall of a blood vessel. Thus, if one maintains a constant pressure, then the reduced equation would predict that the thickness of the vessel wall should vary with the radius of the vessel. In reality, however, pressure within the circulatory system is not constant, indeed it falls off through loss by friction. Yet despite this, for a time the larger and smaller vessels follow the rule laid down by the simplified equation with wall thickness being proportional to vessel size.

### AKG effect

It is known that certain amino acids are metabolised within the wall of the intestine - point out preferential use of AKG to AAs and thus with AKG supplement AA absorption may be enhanced in the bypass rats cf. bypass without AKG.

### Conclusions

The results of this study indicate that a bypass operation of this type affects the elasticity of the arteries to a significant degree.

AKG-intake has a positive effect on the elasticity of the arteries in bypass-operated rats, but not in controls.

Sudden high passive tension has a persistent effect on elasticity of the arteries in both control and bypass-operated rats, making the vessels less able to take sudden pressure changes.

### EXAMPLE 2

### METHODS

The purpose of the study was to elucidate whether the effect observed in the study of example 1 was limited to subject having undergone gastric surgery. This time, experimental subjects that were in need of increased artery elasticity for a more general reason, namely advanced age, were studied.

### Local ethical permission

The study was approved by the Ethical Review Committee for Animal Experiments at Lund University (Ethical allowance M14-05), and was conducted according to European Community regulations concerning the protection of experimental animals.

### Animals and aorta preparation

Female NMRI mice, aged 50 weeks at the start of the trial, were housed at the animal facilities of the Department of Cell & Organism Biology, Lund University, Sweden. The animals were raised under the same conditions with a 12/12 hour light-dark cycle. Mice were fed rodent pellets *ad libitum* (Altromin no.1314 Spezialfutterwerke, Lage, Germany) and given free access to drinking water. Mice were randomly allocated to one of three groups, and fed for 182 days until they had reached 76 weeks of age, at which time they had a body weight of 28 ± 7 g. Mice in Group 1 were fed rodent pellets plus (2% w/v) Na₂-AKG 2 H₂O (n=6), whilst mice in Group 2 were fed rodent pellets plus (2% w/v) Ca-AKG H₂O (n=6). Mice allocated to Group 3 were only fed rodent pellets and were considered to be the Control group (n=6). The level of AKG fed as a supplement to the diet represented 2% of the voluntary feed intake of the mice, which was approx. 10-15% of body weight per day.

The mice were anaesthetized by exposure to 95% CO₂ and killed by cervical dislocation. A dissected portion of the abdominal aorta, prior to the right- and left common iliac arteries, was carefully cleaned to remove adhering tissues. The aorta was cut into approx. 4.5 mm pieces with a diameter at rest of approx.1 mm, and each piece was then securely attached at one end to a force transducer and at the other to a metal pin on a mounting block, as described in part (Harrison et al. Reprod Fertil Dev. 1997;9(7):731-40, Harrison and Flatman Am J Physiol. 1999 Dec;277(6 Pt 2):R1646-53). The weight of the aorta pieces was determined using a scale capable of recording the weight to the nearest 0.01 mg and was on average 2.75 mg.

Aorta sections were immersed into oxygenated and thermostatically controlled chambers (37°C), having an internal depth and diameter of 5.5 and 3.2 cm, respectively, and holding 44 ml of phosphate buffered saline (0.15 M PBS, pH 7.4) comprising in mM; NaCl 136.91, KCl 2.68, Na₂HPO₄ 8.08 and NaH₂PO₄ 1.66. Force was measured using a FTO3 force displacement transducer (Grass Instrument, West Warwick, RI) connected to a home-built bridge amplifier which was interfaced with a 8S PowerLab A/D Converter (ADInstruments, Chalgrove, Oxfordshire, UK). The transducer had a functional range 0-0.05 kg, with a reliable force of 2 mg, equivalent to 0.004% of the functional range. The PowerLab 8S A/D converter was connected to an iBook G4 running Chart v.5.4 Software (AD Instruments, Australia). The data recording was at a sampling speed of 40.000 data samples per second (40 KHz) and the input impedance of the amplifier was 200 MΩ differential

### Force measurements

Aorta sections were suspended vertically, and in duplicate. The recorded signal was adjusted to zero for un-tensioned aorta sections with the aid of an offset dial mounted on the pre-amplifier unit. Each aorta section was then exposed to approx. 5 step-wise increases in tension (each step being approx. 0.09 N), until a final maximal tension of 0.49 N (measured using the FT03 Grass Force transducer) was achieved. This final level of tension was by no means close to the physiological maximum force recorded in aorta sections. The aorta sections were then allowed to relax totally before being exposed to repeat step-wise increases in tension a further two more times, in close succession. Aorta sections were subsequently removed and weighed.

Immediately after a step-wise increase in tension, the recording trace was seen to fall very slightly as the aorta tissue exerted a degree of elastic recoil. This fall in the recording trace was measured over time using the Average Slope calculation available as part of Chart v.5.4 Software (AD Instruments, Australia). Average Slope (µg ms⁻¹) is a time derivative of the data points in a trace selection, and is calculated from the least-square line of best fit.

### Tension calculations

LaPlace's equation assumes the tension (T) in the wall of a hollow cylinder is directly proportional to the cylinders radius (r) and the pressure (p) across the wall, caused by the flow inside the cylinder, such that T = p x r. If one assumes that the tension in the wall of the aorta sections is equivalent to that recorded by the force transducer as the result of a manual stretch, then the pressure that would have given rise to such an increase in tension can be calculated using LaPlace's equation and the measured radius of each aorta section.

Thus the measurement of Average Slope (µg ms⁻¹) obtained for each aorta sample was converted into Newtons (N ms⁻¹) before being adjusted for sample weight to give a final elastic recoil value in N ms⁻¹ mg⁻¹ wet wt.

### Statistical analysis

Data are presented as mean ± SE. Differences between means were tested for statistical significance with the use of a one-way ANOVA and an additional test for Gaussian Normal Distribution. Data were found to be normally distributed and to have equal variance. Differences showing a P value >0.05 were considered non-significant.

### RESULTS

### Tension measurements

Typically, aorta sections were exposed to approx. 0.178 N (0.49 N max. tension / 2.75 mg median tissue wt.) of tension per mg wet weight when fully tensioned. Thus using LaPlace's equation and an average aorta radius of 1.0 mm, the pressure generated was in the order of 0.178 kPa.

An average manual step increase in tension generated 0.09 N (4.95 x 10⁻³ N/mg wet wt.), and aorta sections were typically found to recoil by 0.015 N, a value that represents approximately 16% of the manually applied tension.

### Control mice

The elasticity of the aorta for the controls was 3.3 x 10⁻⁵ ± 7.8 x 10⁻⁷ N ms⁻¹ mg⁻¹ wet wt and 3.4 x 10⁻⁶ ± 9.4 x 10⁻⁷ N ms⁻¹ mg⁻¹ wet wt for the first and second series of stretches, respectively. The repeated stretching protocol resulted in approximately a 90% decrease in elastic recoil; second *versus* first series (Fig 5).

### Na-AKG mice (A)

With Na-AKG-intake, the elasticity of aorta sections was 4.3 x 10⁻⁵ ± 1.6 x 10⁻⁶ N ms⁻¹ mg⁻¹ wet wt and 3.7 x 10⁻⁶ ± 1.1 x 10⁻⁶ N ms⁻¹ mg⁻¹ wet wt for the first and second series of stretches, respectively. Na-AKG-intake also had a significant effect on arterial elasticity compared with the control mice (see Fig 4). The repeated stretching protocol resulted in a 91% decrease in elastic recoil; second *versus* first series (Fig 5).

### Ca-AKG mice (B)

With Ca-AKG-intake, the elasticity of aorta sections was 6.4 x 10⁻⁵ ± 2.7 x 10⁻⁶ N ms⁻¹ mg⁻¹ wet wt and 3.8 x 10⁻⁶ ± 1.2 x 10⁻⁶ N ms⁻¹ mg⁻¹ wet wt for the first and second series of stretches, respectively. Furthermore, Ca-AKG-intake had a significant effect on arterial elasticity compared with the control mice (see Fig 4). The repeated stretching protocol resulted in a 94% decrease in elastic recoil; second *versus* first series (Fig 5.).

### Stretch series and arterial robustness

In all the arteries studied, the initial stretch series (e.g. application of tension followed by relaxation) led to a decrease in elasticity with subsequent applications of tension. This effect may be compared to the sort of damage that would be expected to arise with a sudden increase in blood pressure (see Fig 5).

**Table 1: Robustness of aorta sections to stretch. The number of successful stretches (in triplicate) for excised aorta sections, exposed to 0.49 N max. tension stepwise, without rupture occurring.**

| | **First Replicate** | **Second Replicate** | **Mean (%)** |
|---|---|---|---|
| CONTROL | 4 out of 6 | 4 out of 6 | 66.7 |
| NA-AKG (A) | 4 out of 6 | 5 out of 6 | 75.0 |
| Ca-AKG (B) | 4 out of 6 | 5 out of 6 | 75.0 |

### DISCUSSION

The results of this study clearly show a beneficial effect of alpha-ketoglutarate treatment on arterial elasticity in elderly mice. Moreover, to our knowledge, this is the first time that a therapy capable of targeting the stiffness of large arteries has been reported.

### Animals

The animals in this study were chosen as adults, to have a comparable age to that of an elderly human subject. Upon dissection of the aorta from the mice in this study, it was clear that arterial deposition had taken place such that the aortas appeared almost white to translucent and even after dissection, they retained their tubular shape.

### Blood pressure and tension

In rats aged 6 months and older, a blood pressure, obtained by cannulating the abdominal aorta or the iliac or carotid artery, with a mean of 119 mm Hg was recorded (upper limit 150, and lower limit 92 mm Hg) (Durant, 1927). This author also mentions a correlation between age and blood pressure up until 6 months of age, after which no further increase in blood pressure was recorded, despite a further increase in body weight. This value for blood pressure in a small rodent is very close to that of a resting human subject, where the systolic pressure is normally 120 mm Hg (16 kPa). Moreover, the increases in pressure exerted on the artery sections in the present study represent 1.8 % of that typically found in human arteries, and approximately 1.4 % of that measured in rats (13-14 kPa) (Carroll et al. 2006; Duka et al. 2006).

The aortic media contains sheets of smooth muscle cells, which are tangentially attached to the elastic lamellae; by varying the distribution of force between the elastic and collagenous fibres, changes in smooth muscle tone provide dynamic, or functional regulation of stiffness (McEniery et al. 2007). Indeed at lower levels of arterial pressure, the resulting stress within the aortic wall is taken up predominantly by the elastin fibres, whilst at higher levels of arterial pressure, the stress is generally taken up by the stiffer collagen fibres. Thus one of the effects of ageing is to engage the collagen fibres at lower levels of arterial pressure and concomitantly increase pulse pressure as a result.

The tension developed in an artery depends upon the thickness of the vessel wall, that is to say the amount of connective and muscular tissue comprising the wall. Thus, if one maintains a constant pressure, then Laplace's equation would predict that the thickness of the vessel wall should vary with the radius of the vessel. In reality, however, pressure within the circulatory system is not constant, indeed it falls off through loss by friction. Yet despite this, for a time the larger and smaller vessels follow Laplace's law according to a simplified equation.

The Laplace equation states that the fluid pressure P within a vessel wall is balanced by the tension T of the wall, divided by the radius of curvature r, and the external pressure pₙ, such that P = pₙ + T(1/r). If one chooses to neglect external pressure and any support from surrounding tissues, dealing only with a cylindrical artery of a reasonable size, then the equation can be reduced to P = T / r. In the present study the aorta sections were dissected from the abdominal aorta, prior to the right- and left common iliac arteries, and were of such a diameter that they fulfilled the requirements needed to comply with Laplace's law.

### Ageing arteries

Ageing, which affects organs, tissues and cell types within an organism in different ways, can in many ways be regarded as a differential rate of functional decline (Calabresi et al. 2007). In the vascular wall of large arteries, age-related structural changes occur including stiffening and thickening of the media as well as enlargement of the lumen diameter (Marin & Rodriguez-Martinez, 1999; Dao et al. 2005), and very often these changes are heterogeneous along the arterial tree (Hajdu et al. 1990; Moreau et al. 1998; Laurant et al. 2004). In aorta from aged rats, modification in smooth muscle cell number, increased collagen deposition, and structural alterations of elastin are characteristic features (Jacob, 2003; Dao et al. 2005). Indeed a number of studies report a decline in smooth muscle cell number with age (Cliff, 1970; Orlandi et al. 1993), an increase in collagen type I and III in arteries, and a relative reduction in elastin density (Jacob, 2003; Dao et al. 2005; Marín, 1995).

It is worth noting that the stretch approach adopted in the present study revealed a much weaker degree of elastic recoil in the second *cf.* the first series of repeated stretches. This point highlights and emphasises the ability, or lack thereof, of elderly aorta sections to cope with a period of relatively mild stretch equivalent to an increase in blood pressure of 0.178 kPa. The second stretch may therefore be seen as an index of robustness, which under the present circumstances did not appear to exist in the elderly mouse aorta sections. In man, by the age of 60 years, an average individual has experienced over 2 billion stress cycles of the aorta (average heart rate x age) (McEniery et al. 2007), with damage arising from such stress cycles requiring immediate adjustment and repair involving the elastin, collagen and smooth muscle components of the vessel wall. In the present study, there was no possibility for adjustment to smooth muscle tone, or any chance of repair to elastin and collagen fibres, hence the fact that after the first series of stretches, almost 90 % of the elastic recoil (N ms⁻¹ mg⁻¹ wet wt.) in the Control aortas had been lost, and that similar levels of elastic recoil were found during the second series for the AKG-treated mice, indicates just how vulnerable large vessel recoil is to damage in elderly mice.

### Arterial elastic recoil and AKG

Whilst traditional antihypertensive agents have been reported to reduce arterial stiffness, mostly *via* an indirect effect of lowering mean blood pressure, the relative immunity of peripheral arteries to stiffening with age is usually attributed to a much lower ratio of elastin to smooth muscle and collagen, although it may also reflect other biological processes such as the ability of arteries to remodel themselves (McEniery et al. 2007).

Alpha-ketoglutarate, a rate-determining intermediate in the Krebs cycle, plays a crucial role in cellular energy metabolism. It also functions as a source of glutamate and glutamine, as well as stimulating protein synthesis and inhibiting protein degradation (Hammarqvist et al., 1991). In terms of collagen metabolism, AKG acts not only as a co-factor for prolyl-4-hydrolase which catalyzes the formation of 4-hydroxyproline, essential for the formation of the collagen triple helix, it also contributes to collagen synthesis through an increase in the pool of proline from glutamate (Son et al. 2007).

The better effects in Ca-AKG group as compared to Na-AKG groups can be explained by longer lasting availability of AKG offered in the Ca-AKG salt. The Ca-salt is acting as slow release for the AKG ion controlling its appearance in the intestinal lumen since its solubility is 2 g per 100 ml while the solubility of Na-AKG is 50 times higher. Thus the AKG anion is more rapidly available in the form of Na-AKG. In such situation, a large proportion of the AKG is simply converted to energy when the blood levels are over approximately 10 µg/ml. After enteral administration of Na-AKG blood level of AKG can easily exceed 10 µg/ml. This is never or seldom observed after enteral administration of Ca-AKG. When AKG is offered in the form of Ca-AKG it is released slowed and for a longer time period thus having more time to be converted to proline and other amino-acids instead of energy.

AKG has recently been identified as being a natural ligand for a G-protein-coupled-receptor (CPR99), which is currently known to be expressed in kidney, testis and smooth muscle (He et al., 2004). As a G-protein-coupled receptor ligand, AKG might form a link between TCA-cycle intermediates and both metabolic status and protein/collagen synthesis, indeed this may well prove to be the underlying cause for the observed beneficial effects on aorta wall elasticity seen in the present study.

### CONCLUSION

The results of this study indicate that AKG is effective in improving arterial elasticity not only in subjects that have undergone gastric surgery (example 1) but also other subjects having decreased arterial elasticity. In this case, the subjects were elderly rodents, which are deemed to be a relevant model also for human subjects having decreased arterial elasticity which are also usually aged.

### References

Brolin R.E. Bariatric surgery and long-term control of morbid obesity. JAMA 288: 2793-2796, 2002.
Brolin R.E. Critical analysis of results: weight loss and quality of data. Am.J.Clin.Nutr. 55: 556S-559S, 1992.
Carroll J.F., W.J. Zenebe and T.B. Strange. Cardiovascular function in a rat model of diet-induced obesity. Hypertension 48: 65-72, 2006.
Cliff W.J. The aortic tunica media in aging rats. Exp.Mol.Pathol. 13: 172-189, 1970.
Coates P.S., J.D. Fernstrom, M.H. Fernstrom, P.R. Schauer and S.L. Greenspan. Gastric bypass surgery for morbid obesity leads to an increase in bone turnover and a decrease in bone mass. J.Clin.Endocrinol.Metab. 89: 1061-1065, 2004.
Dao H.H., R. Essalihi, C. Bouvet and P. Moreau. Evolution and modulation of age-related medial elastocalcinosis: impact on large artery stiffness and isolated systolic hypertension. Cardiovasc.Res. 66: 307-317, 2005.
Duka A., I. Duka, G. Gao, S. Shenouda, I. Gavras and H. Gavras. Role of bradykinin B1 and B2 receptors in normal blood pressure regulation. Am.J.Physiol. Endocrionol.Metab. 291: E268-E274, 2006.
Durant R.R. Blood pressure in the rat. Am.J.Physiol. Endocrionol.Metab. 81: 679-685, 1927.
Flanebaum, L. & Belsley, S. J.Gastrointest.Surg. 11, 500-507 (2007).
Flegal K.M., M.D. Carroll, C.L. Ogden and C.L. Johnson. Prevalence and trends in obesity among US adults, 1999-2000. JAMA 288: 1723-1727, 2002.
Foley E.F., P.N. Benotti, B.C. Borlase, J. Hollingshead and G.L. Blackburn. Impact of gastric restrictive surgery on hypertension in the morbidly obese. Am.J.Surg. 163: 294-297, 1992.
Gokce, N. et al. Am.J.Cardiol. 95, 266-268 (2005).
Hajdu M.A., D.D. Heistad, J.E. Siems and G.L. Baumbach. Effects of aging on mechanics and composition of cerebral arterioles in rats. Circ.Res. 66: 1747-1754, 1990.
Harrison, A.P. & Flatman, J.A. Am. J. Physiol., 277, R1646-R1653 (1999).
Jacob M.P. Extracellular matrix remodelling and matrix metalloproteinases in the vascular wall during aging and in pathological conditions. Biomed.Pharmacother. 57: 195-202, 2003.
Kristensen, N.B., Jungvid, H., Fernandez, J.A. & Pierzynowski, S.G. J. Animal Physiol. Animal Nutr. 86, 239-245 (2002).
Laurant P., M. Adrian and A. Berthelot. Effect of age on mechanical properties of rat mesenteric small arteries. Can.J.Physiol.Pharmacol. 82: 269-275, 2004.
Marín J. Age-related changes in vascular responses: a review. Mech.Ageing Dev. 79: 71-114, 1995.
Marín J., M.A. Rodríguez-Martínez. Age-related changes in vascular responses-Exp.Gerontol. 34: 503-512, 1999.
McEniery C.M., I.B. Wilkinson and A.P. Avolio. Age, hypertension and arterial function. Clin.Exp.Pharm.Physiol. 34: 665-671, 2007.
Moreau P., L.V. dÚscio and T.F. Luscher. Structure and reactivity of small arteries in aging. Cardiovasc.Res. 37: 247-253, 1998.
Orlandi A., A. Mauriello, B. Marino and L.G. Spagnoli. Age-related modifications of aorta and coronaries in the rabbit: a morphological and morphometrical assessment. Arch.Gerontol.Geriatr. 17: 37-53, 1993.
Patterson E.J., D.G. Davis, Y. Khajanchee and L.L. Swanström. Comparison of objective outcomes following laparoscopic Nissen fundoplication vs laparoscopic gastric bypass in the morbidly obese with heartburn. Surg.Endosc. 17: 1561-1565, 2003.
Pories, W.J. et al. Annals of Surgery 222, 339-352 (1995).
Son E.D., G.H. Choi, H. Kim, B. Lee, I.S. Chang and J.S. Hwang. Alpha-Ketoglutarate stimulates procollagen production in cultured human dermal fibroblasts, and decreases UVB-induced wrinkle formation following topical application on the dorsal skin of hairless mice. Biol.Pharm.Bull. 30: 1395-1399, 2007.

## Claims

1. A substance selected from the group consisting of:
a. alpha-ketoglutaric acid (AKG) and
b. pharmaceutically acceptable salts of alpha-ketoglutaric acid;
for use in the treatment and/or prophylaxis of hypertension or pulmonary hypertension.

2. A substance for use according to claim 1, wherein the substance is for use in a subject having undergone gastric surgery.

3. A substance for use according to claim 2, wherein the gastric surgery is gastric bypass surgery, gastrectomy, partial gastrectomy or gastric banding.

4. A substance for use according to any of the above claims, wherein the substance is for use in a subject suffering from conditions involving malnutrition.

5. A substance for use to any of the above claims, wherein the substance is for use in a subject being elderly.

6. A substance for use according to any of the above claims, wherein the substance is alpha-ketoglutaric acid or an alkali or alkaline earth metal salt thereof.

7. A substance for use according to claim 6, wherein the substance is sodium alpha-ketoglutarate.

8. A substance for use according to claim 6, wherein the substance is calcium alpha-ketoglutarate.

9. A substance for use according to any of the above claims, wherein the dosage to be given to a patient is in the interval from 1 to 1000 mg/kg body weight/day.

10. A substance for use according to any of the above claims, wherein the dosage to be given to a patient is in the interval from 10 to 400 mg/kg body weight/day.

11. A substance for use according to any of the above claims, wherein the dosage to be given to patients is in the interval from 10 to 100 mg/kg body weight/day.

## Patentansprüche

1. Substanz, ausgewählt aus der Gruppe, die aus Folgendem besteht:
a) α-Ketoglutarsäure (AKG) und
b) pharmazeutisch vertretbaren Salzen von α-Ketoglutarsäure;
zur Verwendung bei der Behandlung und/oder Prophylaxe von Hypertonie oder pulmonaler Hypertonie.

2. Substanz zur Verwendung nach Anspruch 1, wobei die Substanz der Verwendung bei einer Person dient, die sich einer Magenoperation unterzogen hat.

3. Substanz zur Verwendung nach Anspruch 2, wobei es sich bei der Magenoperation um eine Magen-Bypass-Operation, eine Gastrektomie, eine partielle Gastrektomie oder eine bandverstärkte Gastroplastik handelt.

4. Substanz zur Verwendung nach einem der vorangehenden Ansprüche, wobei die Substanz der Verwendung bei einer Person dient, die an Zuständen leidet, die mit Fehlernährung verknüpft sind.

5. Substanz zur Verwendung nach einem der vorangehenden Ansprüche, wobei die Substanz der Verwendung bei einer älteren Person dient.

6. Substanz zur Verwendung nach einem der vorangehenden Ansprüche, wobei es sich bei der Substanz um α-Ketoglutarsäure oder ein Alkali- oder Erdalkalimetallsalz davon handelt.

7. Substanz zur Verwendung nach Anspruch 6, wobei es sich bei der Substanz um Natrium-α-Ketoglutarat handelt.

8. Substanz zur Verwendung nach Anspruch 6, wobei es sich bei der Substanz um Calcium-α-Ketoglutarat handelt.

9. Substanz zur Verwendung nach einem der vorangehenden Ansprüche, wobei die einem Patienten verabreichte Dosierung in dem Intervall von 1 bis 1.000 mg/kg Körpergewicht/Tag liegt.

10. Substanz zur Verwendung nach einem der vorangehenden Ansprüche, wobei die einem Patienten verabreichte Dosierung in dem Intervall von 10 bis 400 mg/kg Körpergewicht/Tag liegt.

11. Substanz zur Verwendung nach einem der vorangehenden Ansprüche, wobei die einem Patienten verabreichte Dosierung in dem Intervall von 10 bis 100 mg/kg Körpergewicht/Tag liegt.

## Revendications

1. Substance choisie dans le groupe constitué par :
a. l'acide alpha-cétoglutarique (AKG) et
b. les sels pharmaceutiquement acceptables de l'acide alpha-cétoglutarique ;
destinée à une utilisation dans le traitement et/ou la prophylaxie de l'hypertension ou de l'hypertension pulmonaire.

2. Substance destinée à une utilisation selon la revendication 1, dans laquelle la substance est destinée à une utilisation chez un sujet ayant subi une chirurgie gastrique.

3. Substance destinée à une utilisation selon la revendication 2, dans laquelle la chirurgie gastrique est une chirurgie de pontage gastrique, une gastrectomie, une gastrectomie partielle ou une gastroplastie par anneau gastrique.

4. Substance destinée à une utilisation selon l'une quelconque des revendications précédentes, dans laquelle la substance est destinée à une utilisation chez un sujet souffrant de pathologies comprenant une malnutrition.

5. Substance destinée à une utilisation selon l'une quelconque des revendications précédentes, dans laquelle la substance est destinée à une utilisation chez un sujet âgé.

6. Substance destinée à une utilisation selon l'une quelconque des revendications précédentes, dans laquelle la substance est l'acide alpha-cétoglutarique ou un sel de métal alcalin ou alcalino-terreux de celui-ci.

7. Substance destinée à une utilisation selon la revendication 6, dans laquelle la substance est l'alpha-cétoglutarate de sodium.

8. Substance destinée à une utilisation selon la revendication 6, dans laquelle la substance est l'alpha-cétoglutarate de calcium.

9. Substance destinée à une utilisation selon l'une quelconque des revendications précédentes, dans laquelle la dose à administrer à un patient se situe dans l'intervalle allant de 1 à 1 000 mg/kg de poids corporel/jour.

10. Substance destinée à une utilisation selon l'une quelconque des revendications précédentes, dans laquelle la dose à administrer à un patient se situe dans l'intervalle allant de 10 à 400 mg/kg de poids corporel/jour.

11. Substance destinée à une utilisation selon l'une quelconque des revendications précédentes, dans laquelle la dose à administrer à un patient se situe dans l'intervalle allant de 10 à 100 mg/kg de poids corporel/jour.
